# EUROPÄISCHE PATENTANMELDUNG

(11) **EP 2 733 479 A1**
(43) Veröffentlichungstag der Anmeldung: **21.05.2014**
(21) Anmeldenummer: 12192739.6
(22) Anmeldetag: 15.11.2012
(51) Int. Cl.: G01N 21/35, G01N 33/28

(54) **Ölmessvorrichtung für elektrisches Antriebsmittel mit Extraktor**

(71) Anmelder: Siemens Aktiengesellschaft, 80333 München (DE)
(72) Erfinder: Buchgraber, Gerhard, 8182 Puch bei Weiz (AT); Atanasova-Höhlein, Ivanka, 90762 Fürth (DE); Kuffel, Maik, 96052 Bamberg (DE); Rehorek, Christian, 90522 Oberasbach (DE); Zafeiris, Konstantinos, 90419 Nürnberg (DE)

(57) **Zusammenfassung**

Die Erfindung betrifft eine Ölmessvorrichtung für ein elektrisches Antriebsmittel mit einem Extraktor (1), wobei der Extraktor (1) eine Ölseite (8) und eine Gasseite (7) aufweist und zum Extrahieren von Gas (5) aus der Ölseite (8) in die Gasseite eingerichtet ist, wobei der Extraktor (1) mindestens eine Infrarot-Messeinrichtung (9) zum Bestimmen mindestens eines Gases (5) in der Gasseite (7) aufweist.

## Beschreibung

Die Erfindung betrifft eine Ölmessvorrichtung für ein elektrisches Antriebsmittel mit einem Extraktor, wobei der Extraktor eine Ölseite und eine Gasseite aufweist und zum Extrahieren von Gas aus der Ölseite in die Gasseite eingerichtet ist, wobei die Gasseite des Extraktors mindestens eine Messeinrichtung zum Bestimmen mindestens eines Gases in der Gasseite aufweist. Die Erfindung betrifft ferner ein ölgefülltes elektrisches Antriebsmittel mit einem Extraktor. Die Erfindung ist insbesondere anwendbar auf Transformatoren.

Sogenannte Schadgassensoren bestehen im Prinzip aus einer Extraktionseinrichtung mit Ölkreislauf und einer Messeinrichtung mit einem Gaskreislauf. Sowohl im Ölkreis als auch im Gaskreis werden Pumpen für eine Zirkulation des Isolieröls bzw. des extrahierten Gasgemischs benötigt. Dabei notwendige Baugruppen wie Ventile, Pumpen und Steuerelektronik sind wie alle technischen Systeme mit einer Fehlerrate behaftet und führen zu einer unerwünscht hohen Ausfallsrate und hohem Wartungsbedarf. Eine technische Lebensdauer solcher Sensoren liegt damit weit unter der technischen Lebensdauer der Primärgeräte, welche der Sensor zu überwachen hat.

Im Isolieröl gelöste Schadgase werden entweder über eine Membran oder eine freie Öloberfläche (Headspace) in den gasförmigen Zustand gebracht und dann mit Hilfe einer Pumpe in einen Detektor zur Messung geleitet.

Unter anderem DIN EN 60599 beschreibt in Betrieb befindliche, mit Mineralöl imprägnierte elektrische Geräte. Beschrieben wird darin insbesondere das Messen von im Öl enthaltenen Gasen und damit als solches die Bestimmung von Basis-Gasquotienten Acetylen (C₂H₂) / Ethen (C₂H₄), Methan (CH₄) / Wasserstoff (H₂) bzw. Ethen (C₂H₄) / Ethan (C₂H₆), um einen Ölzustand zu prüfen bzw. auf Fehler im Betrieb des elektrischen Geräts zu schließen. Dadurch lässt sich auf thermische und elektrische fehlerhafte Betriebszustände schließen. Durch eine Untersuchung des Öls auf einen Gehalt an Kohlenmonoxid, Kohlendioxid und Wasser lässt sich auf eine Zersetzung einer Zelluloseisolierung des elektrischen Geräts schließen.

On-line-Monitoring an Transformatoren wird in Hinsicht auf eine Energieentwicklung (SmartGrid, Offshore) immer wichtiger. Gas-in-Öl-Überwachungssysteme können Fehler erkennen und Trends kontinuierlich aufzeichnen. Derzeit existieren zwei Hauptrichtungen der On-Line-Überwachungs-Philosophie: gemäß einem Ansatz gibt es einfache Systeme, die nur ein Fehlergas bestimmen. Ein-Gas-Überwachungssysteme weisen jedoch nachteiligerweise keine Möglichkeit einer Diagnose auf, wodurch die Anwendungsmöglichkeit eingeschränkt ist. Diese Systeme werden hauptsächlich als Frühwarnsysteme angewendet. Außerdem gibt es Vollsysteme, die eine Gas-in-Öl-Analyse vollständig ersetzen können. Dabei ist nachteilig, dass Vollsysteme teuer in der Anschaffung und kompliziert in Anwendung und Wartung sind.

Bekannt ist zur Gasanalyse allgemein die Verwendung eines Gaschromatographen, mit dem unter Verwendung eines Trägergases aus dem Öl extrahiertes oder aus dem Öl entgastes Gas zur Analyse des Gases verbrannt wird.

Es ist die **Aufgabe** der vorliegenden Erfindung, die Nachteile des Standes der Technik zumindest teilweise zu überwinden. Eine besondere Aufgabe besteht darin, eine Möglichkeit für eine verbesserte Gasanalyse von Öl eines elektrischen Antriebsmittels bereitzustellen.

Diese Aufgabe wird gemäß den Merkmalen der unabhängigen Ansprüche gelöst. Bevorzugte Ausführungsformen sind insbesondere den abhängigen Ansprüchen entnehmbar.

Eine Lösung besteht demgemäß in einer Ölmessvorrichtung für ein elektrisches Antriebsmittel mit einem Extraktor, wobei der Extraktor eine Ölseite und eine Gasseite aufweist und zum Extrahieren von Gas aus der Ölseite in die Gasseite eingerichtet ist, wobei der Extraktor mindestens eine Infrarot-Messeinrichtung zum Bestimmen mindestens eines Gases in der Gasseite aufweist.

Dadurch wird bei einfacherem konstruktivem Aufwand eine qualitativ und quantitativ zuverlässige Gasbestimmung ermöglicht. Insbesondere lässt sich die Zahl der notwendigen Komponenten zur Gasanalyse wesentlich reduzieren. Daraus folgen eine Reduktion der Fehleranfälligkeit und damit eine Steigerung der Zuverlässigkeit und der technischen Lebensdauer der Ölmessvorrichtung. Außerdem wird ein Wartungsaufwand aufgrund der Einfachheit der Komponenten gesenkt.

Unter dem Bestimmen bzw. Detektieren mindestens eines Gases kann insbesondere ein Vorhandensein eines bestimmten Einzelgases und/oder eines bestimmten Gasgemischs und/oder eine quantitative Bestimmung des Einzelgases und/oder Gasgemischs verstanden werden, z.B. in Form einer Konzentration.

Die Gasseite kann auch als ein Gassammelraum aufgefasst werden.

Die Infrarot-Messeinrichtung mag insbesondere mindestens einen Infrarotsensor aufweisen. Die Infrarot-Messeinrichtung mag insbesondere mindestens eine Infrarotlichtquelle aufweisen. Die Arbeitsweise von Infrarot-Messeinrichtungen ist grundsätzlich bekannt und braucht hier nicht weiter ausgeführt zu werden.

Es ist eine Ausgestaltung, dass der Extraktor eine Membran aufweist, welche die Ölseite von der Gasseite trennt. Durch die Membran wird der Gassammelraum auf einer Seite der Membran ausgebildet.

Es ist eine Ausgestaltung, dass ein Gassammelraum eine Messzelle der Infrarot-Messeinrichtung darstellt.

Es ist eine weitere Ausgestaltung, dass die Infrarot-Messeinrichtung innerhalb eines Gassammelraums des Extraktors angeordnet ist. In dem Gassammelraum sind also zumindest Komponenten der Infrarot-Messeinrichtung, insbesondere eine Infrarot-Lichtquelle und ein Infrarot-Sensor einsetzbar, ohne in direkter Berührung mit dem Öl zu stehen. Insbesondere eine der Gas-Infrarot-Messeinrichtung zugeordnete Elektronik kann auch außerhalb des Gassammelraums angeordnet und beispielsweise mit elektrischen Leitern mit innerhalb des Gassammelraums angeordneten Komponenten verbunden sein.

Es ist noch eine weitere Ausgestaltung, dass die Infrarot-Messeinrichtung (insbesondere deren IR-Lichtquelle(n) und deren IR-Sensor(en)) außerhalb eines als Messzelle dienenden Gassammelraums des Extraktors angeordnet ist. Der Gassammelraum mag dazu insbesondere mindestens ein für das Infrarotlicht der Infrarot-Messeinrichtung durchlässiges Fenster aufweisen. Dadurch kann der Gassammelraum besonders einfach ausgebildet werden. Insbesondere mag ganz auf Durchführungen verzichtet werden.

Es ist zudem eine Weiterbildung, dass eine Messzelle der Infrarot-Messeinrichtung von dem Gassammelraum beabstandet angeordnet ist und mit diesem gastechnisch verbunden ist, z.B. über Gasleitungen. Dies ermöglicht eine freie Anordnung der Infrarot-Messeinrichtung.

Auch ist es eine Ausgestaltung, dass die Infrarot-Messeinrichtung Bestandteil eines Infrarot-Spektrometers ist.

Eine weitere Ausgestaltung besteht darin, dass die Infrarot-Messeinrichtung Bestandteil eines Infrarot-Absorptionsspektrometers ist. Mit anderen Worten können vom Grundaufbau her bekannte Infrarot-Spektrometer oder Infrarot-Absorptionsspektrometer verwendet werden.

Es ist eine Ausgestaltung, dass die Infrarot-Messeinrichtung für mehrere unterschiedliche Frequenzbereiche sensitiv ist.

Dadurch können unterschiedliche Gase und/oder Moleküle detektiert werden. Insbesondere ist auch eine gezieltere Bestimmung und Analyse gewünschter Einzelgase bzw. Moleküle realisierbar.

Gemäß einer Weiterbildung ist vorgesehen, dass die Infrarot-Messeinrichtung eine Lichtquelle oder mehrere Lichtquellen, insbesondere Infrarot-Leuchtdioden, aufweist, die auf unterschiedlichen Frequenzen oder Frequenzbändern ausstrahlt ('Breitband-Lichtquelle') bzw. ausstrahlen. Auch kann es gemäß einer Weiterbildung vorgesehen sein, dass die Infrarot-Messeinrichtung einen IR-Sensor bzw. IR-Detektor oder mehrere IR-Sensoren bzw. IR-Detektoren aufweist, welche für unterschiedliche Frequenzen oder Frequenzbänder sensitiv sind.

Die verschiedenen Lichtquellen bzw. Detektoren können je nach Ausgestaltung insbesondere einander überlappende oder nicht überlappende Sensitivitätsbereiche aufweisen.

Insbesondere ist es eine Ausgestaltung, dass die mindestens eine Infrarot-Messeinrichtung oder eine dieser nachgeschaltete Auswerteeinheit zum Detektieren gesättigter Kohlenwasserstoffe ausgebildet ist.

Auch ist es eine Ausgestaltung, dass die mindestens eine Infrarot-Messeinrichtung oder eine dieser nachgeschaltete Auswerteeinheit zum Detektieren ungesättigter Kohlenwasserstoffe ausgebildet ist.

Detektierbar sind insbesondere Kohlendioxid (CO₂), Kohlenmonoxid (CO), Ethin bzw. Acetylen (C₂H₂), Wasserstoff (H₂), Ethen bzw. Ethylen (C₂H₄), Methan (CH₄), Ethan (C₂H₆).

Gemäß einer Weiterbildung ist vorgesehen, dass die Infrarot-Messeinrichtung bzw. die Auswerteeinheit dazu ausgelegt ist, mindestens einen Gas-Summenparameter zu bestimmen. Dadurch wird der Vorteil erreicht, dass sich eine vereinfachte Auswertung von Gasparametern ergibt. Insbesondere ergibt sich so der Vorteil, dass ein zur Diagnose sinnvoll nutzbarer Gasparameter sich selbst dann noch mit einfachen Mitteln in guter Genauigkeit bereitstellen lässt, wenn eine Bestimmung eines Einzelgasparameters schwierig ist, z.B. aufgrund einer vergleichsweise geringen Konzentration eines Einzelgases oder einer Überlagerung von Messsignalen von Einzelgasen. Das Verfahren ist insbesondere auch einsetzbar in Verbindung mit Gasparametern und Gasbestandteilen, welche normgerecht zur Bestimmung von Fehlern einsetzbar sind. Insbesondere ermöglichen diese Gas-Summenparameter eine Bestimmung elektrischer und thermischer Fehler. Mit anderen Worten wird mindestens ein Gas-Summenparameter anstelle eines Einzelgasparameters bestimmt und kann entsprechend zur Diagnose, Fehlersuche usw. des elektrischen Antriebsmittels verwendet werden.

Unter einem Gasparameter kann insbesondere ein zu einem Einzelgas oder zu einem Gasgemisch gehöriger Parameter verstanden werden, z.B. ein bestimmtes Messsignal und/oder mindestens ein daraus abgeleiteter Wert wie eine Gasmenge und/oder eine Konzentration. Unter einem Einzelgasparameter kann insbesondere ein zu einem bestimmten Einzelgas gehöriger Parameter verstanden werden. Unter einem Gas-Summenparameter kann insbesondere ein zu einem bestimmten Gasgemisch gehöriger Parameter verstanden werden.

Eine Ausgestaltung besteht darin, dass die Infrarot-Messeinrichtung bzw. deren Auswerteeinheit dazu ausgelegt ist, mindestens einen Gas-Summenparameter für eine Summe oder Gruppe ungesättigter Kohlenwasserstoffe zu bilden.

Auch ist es eine Ausgestaltung, dass die Infrarot-Messeinrichtung bzw. deren Auswerteeinheit dazu ausgelegt ist, mindestens einen Gas-Summenparameter für eine Summe oder Gruppe gesättigter Kohlenwasserstoffe zu bilden. Diese können besonders einfach zusammen bestimmt bzw. ausgewertet werden. Jedoch sind auch Mischformen möglich, z.B. dass durch die Infrarot-Messeinrichtung bzw. deren Auswerteeinheit der Gas-Summenparameter für eine Summe oder Gruppe gesättigter und ungesättigter Kohlenwasserstoffe gebildet wird. Diese können besonders einfach zusammen bestimmt bzw. ausgewertet werden.

Noch eine Ausgestaltung besteht darin, dass durch die Infrarot-Messeinrichtung bzw. deren Auswerteeinheit zusätzlich mindestens ein Einzelgasparameter bestimmt wird, insbesondere Kohlendioxid (CO₂), Kohlenmonoxid (CO), Ethin bzw. Acetylen (C₂H₂), Wasserstoff (H₂), Ethen bzw. Ethylen (C₂H₄), Methan (CH₄) und/oder Ethan (C₂H₆).

Noch eine Ausgestaltung besteht darin, dass durch die Infrarot-Messeinrichtung bzw. deren Auswerteeinheit ein Quotient oder Verhältnis von Gasparametern bestimmt wird, wobei mindestens eine Größe des Verhältnisses ein Gas-Summenparameter ist. Das Verhältnis kann beispielsweise dazu verwendet werden, um eine oder mehrere Auswertungen, Diagnosen und/oder Aktionen mit noch höherem Aussagewert durchzuführen als z.B. bei einer Schwellwertbetrachtung absoluter Größen. Das Verhältnis mag beispielsweise ein Verhältnis von Einzelgasparametern und Gas-Summenparametern und/oder ein Verhältnis von mehreren Gas-Summenparametern umfassen.

Es ist eine Weiterbildung davon, dass durch die Infrarot-Messeinrichtung bzw. deren Auswerteeinheit ein Verhältnis aus einem Gas-Summenparameter ungesättigter Kohlenwasserstoffe und aus einem Gas-Summenparameter gesättigter Kohlenwasserstoffe gebildet wird, insbesondere zur Bestimmung eines thermischen Fehlers. Beispielsweise kann dazu ein Verhältnis bestimmt werden aus Gas-Summenparametern der Form (ΣCₙH₂ₙ / ΣCₙH₂ₙ₊₂), insbesondere umfassend C₂H₄ und C₂H₆ (n=2). Mit anderen Worten mögen insbesondere zueinander verschiedene Gas-Summenparameter bestimmt werden, welche einerseits aus der Gruppe der ungesättigten Kohlenwasserstoffe und andererseits aus der Gruppe der gesättigten Kohlenwasserstoffe genommen werden.

Eine Weiterbildung dazu ist, dass durch die Infrarot-Messeinrichtung bzw. deren Auswerteeinheit zusätzlich ein Verhältnis aus einem Gas-Summenparameter gesättigter Kohlenwasserstoffe und einem Einzelgasparameter, z.B. Kohlenmonoxid, gebildet, insbesondere ausgewertet, wird, insbesondere zur Bestimmung eines thermischen Fehlers. Beispielhaft kann ein Quotient der Form (ΣCₙH₂ₙ₊₂ / CO) bestimmt werden. Bestimmbar sind dadurch insbesondere thermische Fehler von mit Mineralöl imprägnierten elektrischen Geräten, insbesondere unter Einsatz einer Infrarot-Sensorik. Insbesondere wird Kohlenmonoxid nicht lediglich zur Bestimmung eines Zelluloseabbaus verwendet.

Es ist auch eine Weiterbildung, dass durch die Infrarot-Messeinrichtung bzw. deren Auswerteeinheit ein Verhältnis aus Acetylen und einem Gas-Summenparameter gesättigter Kohlenwasserstoffe gebildet und ausgewertet wird, insbesondere zur Bestimmung eines elektrischen Fehlers. Dazu wird insbesondere ein Quotient der Form (C₂H₂ / ΣCₙH₂ₙ₊₂), insbesondere umfassend C₂H₆ (n=2), verwendet.

Eine weitere Weiterbildung dazu besteht darin, dass durch die Infrarot-Messeinrichtung bzw. deren Auswerteeinheit zusätzlich ein Gas-Summenparameter ungesättigter Kohlenwasserstoffe und ein Quotient aus Acetylen und einem Gas-Summenparameter gesättigter Kohlenwasserstoffe ausgewertet wird, insbesondere zur Bestimmung eines elektrischen Fehlers. Dies ist insbesondere realisierbar in der Form (ΣCₙH₂ₙ + C₂H₂ / ΣC₂H₂ₙ₊₂). Bestimmbar sind somit auch elektrische Fehler von mit Mineralöl imprägnierten elektrischen Geräten, speziell unter Einsatz einer Infrarot-Sensorik.

Noch eine weitere Weiterbildung besteht darin, dass durch die Infrarot-Messeinrichtung bzw. deren Auswerteeinheit ein Quotient aus Kohlendioxid und Kohlenmonoxid gebildet und ausgewertet wird zur Bestimmung eines Zustands einer festen Isolierung, insbesondere eines Zelluloseabbaus. Dies erfolgt insbesondere in der Form (CO₂ / CO).

Eine weitere Lösung der Aufgabe besteht in einem insbesondere mit Öl gefüllten elektrischen Antriebsmittel mit einem derart ausgebildeten Extraktor.

Das ölgefüllte Antriebsmittel kann gemäß einer Weiterbildung ein Transformator sein. Das Öl ist insbesondere darin als ein Isoliermittel und ggf. Kühlmittel eingesetzt. Das ölgefüllte Antriebsmittel kann gemäß einer anderen Weiterbildung ein Motor sein. Hier mag das Öl auch als ein Schmiermittel eingesetzt sein.

Eine Weiterbildung besteht darin, dass das Antriebsmittel bzw. die Ölmessvorrichtung einen Ölkreislauf aufweist, in welchem sich das Öl befindet.

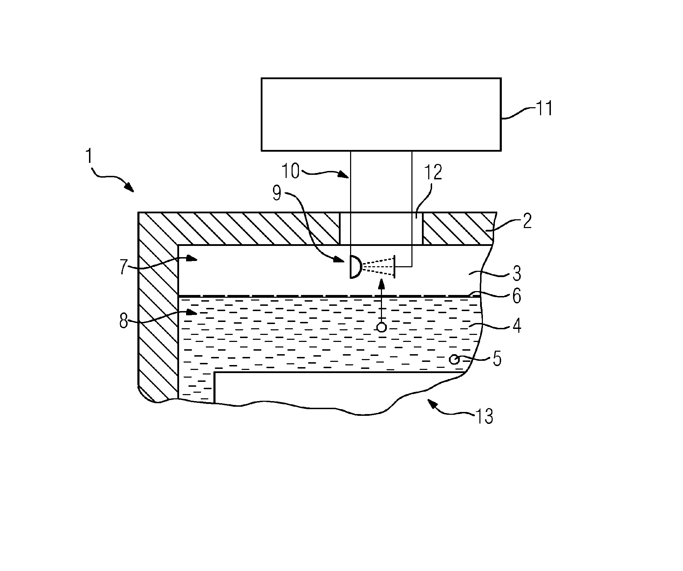

In der folgenden Figur wird die Erfindung anhand eines Ausführungsbeispiels schematisch genauer beschrieben. Die Figur zeigt in Seitenansicht teilweise geschnitten Komponenten einer Ölmessvorrichtung für ein elektrisches Antriebsmittel in Form eines Transformators mit einem Extraktor und einer Infrarot-Messanordnung.

Die **Fig.** zeigt einen Ausschnitt eines Extraktors 1 eines Transformators 13. In einem Gehäuse 2 des Extraktors 1 befinden sich ein Gassammelraum 3 und ein Bereich mit Öl 4, welches insbesondere als Schmiermittel, Kühlmittel und/oder Isolierflüssigkeit verwendet wird.

Eine durch das Gehäuse 2 führende Membran 6 trennt eine Gasseite 7 mit dem Gassammelraum 3 und eine Ölseite 8 mit dem Bereich mit dem Öl 4 voneinander. Gas 5, welches sich auf der Ölseite 8 im Öl 4 befindet und mehrere Einzelgase oder Gaskomponenten aufweist, tritt durch die Membran 6 in die Gasseite 7 über und sammelt sich im Gassammelraum 3.

In dem Gassammelraum 3 ist eine Messkomponente einer Infrarot-Messeinrichtung 9 angeordnet. Der Gassammelraum 3 dient also auch als eine Messzelle. Die beispielhafte Messkomponente besteht aus einer Infrarot-Lichtquelle und einem davon beabstandeten Infrarot-Sensor bzw. -Detektor. Zwischen der Infrarot-Lichtquelle und dem Infrarot-Detektor befindet sich ein Zwischenraum, in den das in den Gassammelraum 3 eingetretene Gas 5 gelangt und dort ausgemessen wird.

Über elektrische Leitungen 10 sind die Komponenten mit einer Auswerteeinheit 11 verbunden. Insbesondere ist die Auswerteeinheit 11 als eigenständiger Prozessor ausgestaltet oder Bestandteil eines übergeordneten Vorrichtungsprozessors, welcher auch zur Steuerung anderer Funktionalitäten der Vorrichtung ausgelegt ist. Eine Öffnung 12 zum Durchführen der elektrischen Leitungen 10 kann insbesondere gasdicht verschlossen sein. Die Öffnung 12 kann aber auch offen sein oder mit einer Leitung zum Abführen gesammelten Gases verbunden sein.

Die Auswerteeinheit 11 ist hier dazu ausgelegt, zumindest aus von der Infrarot-Messeinrichtung 9 gemessenen Messwerten mindestens einen Gas-Summenparameter zu bestimmen, z.B. eine Konzentration. Insbesondere ist die Auswerteeinheit 11 ausgelegt, im Rahmen einer Auswertung zur Bestimmung von Fehlern usw. des Transformators 13 Quotienten von Gas-Summenparametern zu bilden und ggf. auszuwerten.

Für die Quotientenbildung oder Verhältnisbildung werden insbesondere gesättigte gegenüber ungesättigten Kohlenwasserstoffen bzw. ungesättigte gegenüber gesättigten Kohlenwasserstoffen betrachtet. Zur Bestimmung eines thermischen Fehlers wird insbesondere ein Quotient aus einer Gruppe ungesättigter Kohlenwasserstoffe zu einer Gruppe gesättigter Kohlenwasserstoffe betrachtet. Zur Bestimmung eines thermischen Fehlers kann zusätzlich auch eine Quotientenbildung aus einer Gruppe gesättigter Kohlenwasserstoffe zu Kohlenmonoxid betrachtet werden.

Zur Bestimmung eines elektrischen Fehlers kann insbesondere ein Quotient aus Acetylen zu einer Gruppe gesättigter Kohlenwasserstoffe betrachtet werden. Zur zusätzlichen Bestimmung eines elektrischen Fehlers mag auch eine Gruppe ungesättigter Kohlenwasserstoffe zuzüglich Acetylen im Verhältnis zu einer Gruppe gesättigter Kohlenwasserstoffen betrachtet werden.

Zur Bestimmung eines Zelluloseabbaus wird insbesondere ein Quotient aus Kohlendioxid zu Kohlenmonoxid betrachtet.

Über- bzw. Unterschreiten die Gas-Summenparameter und/oder Verhältnisse bzw. Quotienten davon bestimmte Schwellwerte, so dient dies z.B. als Warnung oder Hinweis auf einen möglichen Fehler oder Wartungsfall. Gegebenenfalls kann die Auswerteeinheit 11 auch dazu ausgelegt sein, neben der Ausgabe von Warnungen aktiv in einen laufenden Prozess einzugreifen, z.B. in den Betrieb des Transformators 13.

Eine Diagnoseausgabe seitens der Auswerteeinheit 11 erfolgt insbesondere dann, wenn bestimmte Mindestwerte erreicht werden. Beispielsweise kann als Mindestwert für Kohlenmonoxid ≥ 20 ppm angesetzt werden. Als Bedingung für eine Ausgabe eines Hinweises, einer Warnung usw. im Fall eines Gas-Summenwerts ungesättigter Kohlenwasserstoffe kann z.B. als Bedingung ≥ 10 ppm angesetzt werden.

Selbstverständlich ist die vorliegende Erfindung nicht auf das gezeigte Ausführungsbeispiel beschränkt.

Anstelle eines Gehäuses 2 des Extraktors 1, welches auch Komponenten des elektrischen Antriebsmittels 13 aufnimmt, sind insbesondere auch Anordnungen möglich, bei denen der Extraktor 1 ein eigenständiges Gehäuse 2 hat, welches über Leitungen, insbesondere Ölleitungen in einen Ölkreislauf auf der Ölseite 8 eingebunden ist. Solche Ölleitungen führen dann aus einem Innenraum eines elektrischen Antriebsmittels, beispielsweise eines Transformators, in das Gehäuse des Extraktors und insbesondere aus dem Gehäuse des Extraktors wieder zurück in den Innenraum des elektrischen Antriebsmittels.

Auch braucht eine Trennung der Ölseite 8 von der Gasseite 7 mittels der Membran 6 nicht zwingend in der skizzierten Art und Weise erfolgen. Ggf. kann die Membran ganz entfallen, wenn z.B. Gas aus einer offenen Öloberfläche in einen darüber liegenden Gassammelraum austritt. Wichtig für die insbesondere erste Ausgestaltung ist insbesondere, dass der Extraktor 1 die Gasseite 7 aufweist, in welcher z.B. Komponenten der Infrarot-Messeinrichtung 9 angeordnet sind, so dass in der Gasseite 7 befindliche Gase bestimmbar und mittels der Auswerteeinheit analysierbar sind.

Anstelle lediglich einer Infrarot-Leuchteinrichtung, insbesondere Infrarot-Leuchtdiode und eines Infrarot-Sensors bzw. Infrarot-Detektors können auch zusätzliche Komponenten der Infrarot-Messeinrichtung 11 innerhalb der Gasseite 7 bzw. des Gassammelraums 3 angeordnet sein. Insbesondere kann auch die Auswerteeinheit 11 ganz oder teilweise innerhalb der Gasseite 7 angeordnet sein. Alternativ mag die Infrarot-Messeinrichtung 9 auch ganz außerhalb des Gassammelraums 3 angeordnet sein.

Als weitere Auswertekriterien, insbesondere zur Quotientenbildung können auch weitere Einzelgase oder summierte Gase bzw. Moleküle mittels insbesondere der Infrarot-Messeinrichtung 9 zur Auswertung herangezogen werden.

Die Verwendung eines zusätzlichen Sensors, z.B. Wasserstoffsensors, kann zur weiteren Präzisierung der Diagnose eingesetzt werden.

## Patentansprüche

1. Ölmessvorrichtung für ein elektrisches Antriebsmittel (13) mit einem Extraktor (1), wobei der Extraktor (1) eine Ölseite (8) und eine Gasseite (7) aufweist und zum Extrahieren von Gas (5) aus der Ölseite (8) in die Gasseite eingerichtet ist, **dadurch gekennzeichnet, dass** der Extraktor (1) mindestens eine Infrarot-Messeinrichtung (9) zum Bestimmen mindestens eines Gases (5) in der Gasseite (7) aufweist.

2. Ölmessvorrichtung nach Anspruch 1, bei welcher der Extraktor eine Membran (6) aufweist, welche die Ölseite (8) von der Gasseite (7) trennt.

3. Ölmessvorrichtung nach einem der vorhergehenden Ansprüche, bei der die Infrarot-Messeinrichtung (9) innerhalb eines Gassammelraums (3) des Extraktors angeordnet ist.

4. Ölmessvorrichtung, bei der die Infrarot-Messeinrichtung (9) außerhalb eines Gassammelraums (3) des Extraktors angeordnet ist.

5. Ölmessvorrichtung nach einem der vorhergehenden Ansprüche, bei der die Infrarot-Messeinrichtung (9) Bestandteil eines Infrarot-Spektrometers ist.

6. Ölmessvorrichtung nach einem der vorhergehenden Ansprüche, bei der die Infrarot-Messeinrichtung (9) Bestandteil eines Infrarot-Absorptionsspektrometers ist.

7. Ölmessvorrichtung nach einem der vorhergehenden Ansprüche, bei der die Infrarot-Messeinrichtung (9) für mehrere unterschiedliche Frequenzbereiche sensitiv ist.

8. Ölmessvorrichtung nach einem der vorhergehenden Ansprüche, bei der die mindestens eine Infrarot-Messeinrichtung (9) oder eine dieser nachgeschaltete Auswerteeinheit (11) zum Detektieren gesättigter Kohlenwasserstoffe ausgebildet ist.

9. Ölmessvorrichtung nach einem der vorhergehenden Ansprüche, bei der die mindestens eine Infrarot-Messeinrichtung (9) oder eine dieser nachgeschaltete Auswerteeinheit (11) zum Detektieren ungesättigter Kohlenwasserstoffe ausgebildet ist.

10. Ölgefülltes elektrisches Antriebsmittel (13), insbesondere Transformator, mit einem Extraktor (1) einer Ölmessvorrichtung nach einem der vorstehenden Ansprüche.
